# EUROPEAN PATENT APPLICATION

(11) **EP 4 702 924 A1**
(43) Date of publication of application: **04.03.2026**
(21) Application number: 24796330.9
(22) Date of filing: 28.04.2024
(51) Int. Cl.: A61B 5/283, A61B 5/352

(54) **MEDICAL INSTRUMENT**

(30) Priority: 28.04.2023 CN 202310483658
(71) Applicant: United Innomed (Shanghai) Limited, Shanghai 201315 (CN)
(72) Inventor: WANG, Li, Shanghai 201315 (CN); SUN, Qingyi, Shanghai 201315 (CN)
(74) Representative: Barker Brettell LLP
(86) International application number: PCT/CN2024/090431
(87) International publication number: WO 2024/222961

(57) **Abstract**

Disclosed in the present application is a medical instrument, which comprises a control device that is configured to execute the following step during an operation period: according to the difference value between a first specific time and a second specific time, determining a ventricular activation source of the heartbeat. According to the difference value between the first specific time and the second specific time, the present application determines the ventricular activation source of the heartbeat without the need of using time information of atriums and also without the need of waiting for a complete ventricular activation of the heart to finish, and moreover, without the need of using a morphological algorithm. In addition, the present application involves a simple classification mode and thus does not affect the energy consumption of medical instruments.

## Description

The present application claims the priority to Chinese Patent Application CN202310483658.1, filed on April 28, 2023. The contents of the Chinese patent application are incorporated herein by reference in its entireties.

### TECHNICAL FIELD

The present application relates to the technical field of medical instruments, and in particular to a medical instrument.

### BACKGROUND

In medical instruments that provide cardiac therapy, it is important to determine whether an origin of ventricular activation is atrium originated ventricular activation or ventricle originated ventricular activation. It is clinically important for arrhythmia diagnosis, therapy, etc. to determine whether a particular ventricular beat or a group of beats is sinus rhythm (SR), a premature atrial contraction (PAC), or a premature ventricular contraction (PVC), or whether ventricular pacing causes ventricular activation. Therefore, for various clinical applications, a method for automatically classifying the origin of ventricular activation of each heartbeat as either atrium originated ventricular activation or ventricle originated ventricular activation is important.

In general, in contrast to periods during which sinus rhythm is relatively stable, the temporal information of the current ventricular activation relative to a previous activation (R-R interval) in combination with the information about the morphology of the cardiac electrical activity of that particular activation is used to identify a premature beat and its source (atrial or ventricular). In particular, the duration of the ventricular activation (i.e., the R-wave width) is used to distinguish PVCs from sinus beats or PACs. In contrast to the abnormal (wider) morphology usually seen in the ventricle originated ventricular activation (e.g., PVCs), the atrium originated ventricular activation typically exhibits an R-wave morphology similar to that during sinus rhythm. In existing ICDs (implantable cardioverter defibrillators), distinguishing between VT (ventricular tachycardia) and SVT (supraventricular tachycardia) in regions with similar high ventricular rates is of great significance, and typically requires a larger R-wave width or a different morphology than during sinus rhythm.

However, in the automatic classification using, for example, R-wave width or morphology, not only special hardware and relatively cumbersome computations are required for far-field electrocardiograph signals, but the computation must not be performed until the ventricular activation is completed. This is the case in ICD, for example. On the other hand, in some medical instruments and clinical applications, R-wave width or morphology cannot be calculated in some cases, such as when no electrocardiograph (ECG) or far-field intracardiac electrogram (EGM) is available. In addition, if treatment is required prior to the completion of ventricular activation, for example in the case of cardiac contractility modulation (CCM) treatment, the R-wave width method is not always relied on due to timing considerations.

Several methods exist in the prior art for capture confirmation or verification in a pacing system, including evoked potentials, impedance, etc. The ability to confirm capture allows a pacing threshold to be automatically measured and/or monitored over time according to which the pacing system outputs sufficient suitable energy to ensure the capture while saving energy to remain valid, safe and efficient over time, minimizing the need for surgeon intervention. The confirmation of the capture often requires the addition of special hardware and software, increasing the complexity of a device.

### CONTENT OF THE PRESENT INVENTION

The present application provides a medical instrument, including a control device configured to perform, during an operational period, the following step:
determining the origin of ventricular activation of a heartbeat according to a difference value between a first specific time and a second specific time;
where the first specific time and the second specific time are selected from the following times acquired within the operational period: sensing time (LS1_OP) corresponding to a first sensed event in a first in-vivo near-field myocardial electrogram acquired on the basis of a first electrode pair, time when a pacing pulse is delivered through the first electrode pair (VP_OP), sensing time (GS_OP) of an R-wave in a far-field cardiac electrogram, sensing time (LS2_OP) corresponding to a second sensed event in a second in-vivo near-field myocardial electrogram acquired on the basis of a second electrode pair, and sensing time (LS2'_OP) of ventricular muscle activation corresponding to a pacing event in the second in-vivo near-field myocardial electrogram acquired by the second electrode pair.

Optionally, the control device is further configured to perform, during the operational period, the following steps:
acquiring the first in-vivo near-field myocardial electrogram by the first electrode pair; and
acquiring a first sensing time (GS_OP) and a second sensing time (LS1_OP); where the first sensing time is the first specific time, and the first sensing time is a sensing time (GS_OP) of an R-wave in a far-field cardiac electrogram; the second sensing time is the second specific time, and the second sensing time is the sensing time (LS1_OP) corresponding to the sensed event in the first in-vivo near-field myocardial electrogram; and the R-wave in the far-field cardiac electrogram and the sensed event in the first in-vivo near-field myocardial electrogram correspond to the same heartbeat; and
the step of determining the origin of ventricular activation of a heartbeat according to a difference value between a first specific time and a second specific time includes:
   determining the origin of ventricular activation of the heartbeat according to a difference value between the first sensing time (GS_OP) and the second sensing time (LS1_OP); where the origin of ventricular activation includes atrium originated ventricular activation and ventricle originated ventricular activation.

Optionally, the step of determining the origin of ventricular activation of the heartbeat according to a difference value between the first sensing time (GS_OP) and the second sensing time (LS1_OP) includes in particular:
if a difference value between a first difference value and a second difference value is greater than a first threshold, determining that the origin of ventricular activation of the heartbeat is ventricle originated ventricular activation, otherwise determining that the origin of ventricular activation of the heartbeat is atrium originated ventricular activation;
where the first difference value is the difference value between the first sensing time (GS_OP) and the second sensing time (LS1_OP), and the second difference value is a difference value between a third sensing time (GS_SUP) and a fourth sensing time (LS1_SUP); the third sensing time (GS_SUP) is a sensing time of an R-wave in a far-field cardiac electrogram acquired within a set up period, and the fourth sensing time (LS1_SUP) is a sensing time corresponding to a sensed event in an in-vivo near-field myocardial electrogram acquired by the first electrode pair within the set up period; and the origin of ventricular activation of the heartbeat corresponding to the set up period is atrium originated ventricular activation.

Optionally, the second difference value is determined from difference values between the third sensing times (GS_SUP) and the fourth sensing times (LS1_SUP) corresponding to a plurality of heartbeats.

Optionally, the step of determining the origin of ventricular activation of the heartbeat according to a difference value between the first sensing time and the second sensing time includes in particular:
if the difference value between the first sensing time (GS_OP) and the second sensing time (LS1_OP) is greater than a second threshold, determining that the origin of ventricular activation of the heartbeat is ventricle originated ventricular activation, otherwise determining that the origin of ventricular activation of the heartbeat is another ventricular activation.

Optionally, the control device is further configured to perform, during the operational period, the following step:
acquiring the second in-vivo near-field myocardial electrogram by the second electrode pair; and
the step of determining the origin of ventricular activation of the heartbeat according to a difference value between the first sensing time (GS_OP) and the second sensing time (LS1_OP) includes in particular:
   determining the origin of ventricular activation of the heartbeat according to the difference value between the first sensing time (GS_OP) and the second sensing time (LS1_OP) and a difference value between the first sensing time (GS_OP) and a fifth sensing time (LS2_OP);
   where the fifth sensing time (LS2_OP) is the sensing time corresponding to the sensed event in the second in-vivo near-field myocardial electrogram, and the sensed event in the second in-vivo near-field myocardial electrogram and the R-wave in the far-field cardiac electrogram correspond to the same heartbeat.

Optionally, the control device is further configured to perform, during the operational period, the following steps:
acquiring a first in-vivo near-field myocardial electrogram by the first electrode pair and acquiring a second sensing time (LS1_OP); where the second sensing time is the first specific time, and the second sensing time is a sensing time corresponding to a sensed event in the first in-vivo near-field myocardial electrogram; and
acquiring a second in-vivo near-field myocardial electrogram by the second electrode pair and acquiring a fifth sensing time (LS2_OP); where the fifth sensing time is the second specific time, the fifth sensing time is a sensing time corresponding to a sensed event in the second in-vivo near-field myocardial electrogram, and the sensed event in the second in-vivo near-field myocardial electrogram and the sensed event in the first in-vivo near-field myocardial electrogram correspond to the same heartbeat; and
the step of determining the origin of ventricular activation of a heartbeat according to a difference value between a first specific time and a second specific time includes:
   determining the origin of ventricular activation of the heartbeat according to a difference value between the second sensing time (LS1_OP) and the fifth sensing time (LS2_OP); where the origin of ventricular activation includes atrium originated ventricular activation and ventricle originated ventricular activation.

Optionally, the step of determining the origin of ventricular activation of the heartbeat according to a difference value between the second sensing time and the fifth sensing time includes in particular:
if a difference value between a third difference value and a fourth difference value is greater than a third threshold, determining that the origin of ventricular activation of the heartbeat is ventricle originated ventricular activation, otherwise determining that the origin of ventricular activation of the heartbeat is atrium originated ventricular activation;
where the third difference value is the difference value between the second sensing time (LS1_OP) and the fifth sensing time (LS2_OP), and the fourth difference value is determined from a difference value between a fourth sensing time (LS1_SUP) and a sixth sensing time (LS2_SUP); the fourth sensing time (LS1_SUP) is a sensing time corresponding to a sensed event in an in-vivo near-field myocardial electrogram acquired by the first electrode pair within a set up period, and the sixth sensing time is a sensing time (LS2_SUP) corresponding to a sensed event in an in-vivo near-field myocardial electrogram acquired by the second electrode pair within the set up period; and the origin of ventricular activation of the heartbeat corresponding to the set up period is atrium originated ventricular activation.

Optionally, the fourth difference value is determined from difference values between the fourth sensing times (LS1_SUP) and the sixth sensing times (LS2_SUP) corresponding to a plurality of heartbeats.

Optionally, the step of determining the origin of ventricular activation of the heartbeat according to a difference value between the second sensing time (LS1_OP) and the fifth sensing time (LS2_OP) includes in particular: if the difference value between the second sensing time (LS1_OP) and the fifth sensing time (LS2_OP) is greater than a fourth threshold, determining that the origin of ventricular activation of the heartbeat is ventricle originated ventricular activation, otherwise determining that the origin of ventricular activation of the heartbeat is another ventricular activation.

Optionally, the control device is further configured to perform, during the operational period, the following step: determining a length of a R-wave sensing time window corresponding to the R-wave in the far-field cardiac electrogram acquired within the set up period from the origin of ventricular activation of the heartbeat.

Optionally, the control device is further configured to perform, during the operational period, the following step:
acquiring a first sensing time (GS_OP) and a first delivery time (VP_OP); where the first sensing time (GS_OP) is the second specific time, the first sensing time is a sensing time of an R-wave in a far-field cardiac electrogram, the first delivery time (VP_OP) is the first specific time, and the first delivery time is time when a pacing pulse is delivered through the first electrode pair; and
the step of determining the origin of ventricular activation of a heartbeat according to a difference value between a first specific time and a second specific time includes:
   determining whether the ventricle is captured from a difference value between the first delivery time (VP_OP) and the first sensing time (GS_OP).

Optionally, the step of determining whether the ventricle is captured from a difference value between the first delivery time and the first sensing time includes in particular: if a difference value between a fifth difference value and a sixth difference value is less than a fifth threshold, determining the ventricle is captured, otherwise determining the ventricle is not captured;
where the fifth difference value is the difference value between the first delivery time (VP_OP) and the first sensing time (GS_OP), and the sixth difference value is determined from a difference value between a second delivery time (VP_SUP) and a seventh sensing time (GS'_SUP); and the seventh sensing time is a sensing time (GS'_SUP) of an R-wave in a far-field cardiac electrogram acquired during the ventricle is captured by pacing within a set up period, and the second delivery time is time (VP_SUP) when a pacing pulse is delivered through the first electrode pair during the ventricle is captured by pacing within the set up period.

Optionally, the sixth difference value is determined from difference values between the second delivery times (VP_SUP) and the seventh sensing times (GS'_SUP) corresponding to a plurality of heartbeats.

Optionally, the step of determining whether the ventricle is captured from a difference value between the first delivery time (VP_OP) and the first sensing time (GS_OP) includes in particular: if the difference value between the first delivery time (VP_OP) and the first sensing time (GS_OP) is a positive number and is less than a sixth threshold, determining the ventricle is captured, otherwise determining the ventricle is not captured.

Optionally, the control device is further configured to perform, during the operational period, the following steps:
acquiring a first delivery time (VP_OP); where the first delivery time (VP_OP) is the first specific time, and the first delivery time is time when a pacing pulse is delivered through the first electrode pair; and
acquiring the second in-vivo near-field myocardial electrogram by the second electrode pair and acquiring an eighth sensing time (LS2'_OP); where the eighth sensing time is the second specific time, and the eighth sensing time is a sensing time (LS2'_OP) of ventricular muscle activation corresponding to a pacing event in the second in-vivo near-field myocardial electrogram; and
the step of determining the origin of ventricular activation of a heartbeat according to a difference value between a first specific time and a second specific time includes:
determining whether the ventricle is captured from a difference value between the first delivery time (VP_OP) and the eighth sensing time (LS2'_OP).

Optionally, the step of determining whether the ventricle is captured from a difference value between the first delivery time (VP_OP) and the eighth sensing time (LS2'_OP) includes in particular: if a ratio of a seventh difference value to an eighth difference value is greater than a seventh threshold, determining the ventricle is captured, otherwise determining the ventricle is not captured;
where the seventh difference value is the difference value between the first delivery time (VP_OP) and the eighth sensing time (LS2'_OP), and the eighth difference value is determined from a difference value between a fourth sensing time (LS1_SUP) and a sixth sensing time (LS2_SUP); and the sixth sensing time (LS2_SUP) is a sensing time corresponding to a sensed event in the second in-vivo near-field myocardial electrogram acquired by the second electrode pair within a set up period, the fourth sensing time (LS1_SUP) is a sensing time corresponding to a sensed event in the first in-vivo near-field myocardial electrogram acquired by the first electrode pair within the set up period, and the origin of ventricular activation of the heartbeat corresponding to the set up period is atrium originated ventricular activation.

Optionally, the step of determining whether the ventricle is captured from a difference value between the first delivery time and the eighth sensing time includes in particular: if a difference value between a seventh difference value and a ninth difference value is less than an eighth threshold, determining the ventricle is captured, otherwise determining the ventricle is not captured;
where the seventh difference value is the difference value between the first delivery time (VP_OP) and the eighth sensing time (LS2'_OP), and the ninth difference value is determined from a difference value between a second delivery time (VP_SUP) and a ninth sensing time (LS2'_SUP); and the ninth sensing time (LS2'_SUP) is a sensing time of ventricular muscle activation corresponding to a pacing event in the second in-vivo near-field myocardial electrogram acquired by the second electrode pair during the ventricle is captured by spacing within a set up period, and the second delivery time (VP_SUP) is time when a pacing pulse is delivered through the first electrode pair during the ventricle is captured by pacing within the set up period.

Optionally, the control device is further configured to perform, during the operational period, the following steps:
acquiring a first sensing time (GS_OP); where the first sensing time is the first specific time, and the first sensing time is a sensing time of an R-wave in a far-field cardiac electrogram; and
acquiring the second in-vivo near-field myocardial electrogram by the second electrode pair and acquiring an eighth sensing time (LS2'_OP); where the eighth sensing time is the second specific time, and the eighth sensing time is a sensing time of ventricular muscle activation corresponding to a pacing event in the second in-vivo near-field myocardial electrogram; and
the step of determining the origin of ventricular activation of a heartbeat according to a difference value between a first specific time and a second specific time includes:
   determining whether the ventricle is captured from a difference value between the eighth sensing time (LS2'_OP) and the first sensing time (GS_OP).

Optionally, the step of determining whether the ventricle is captured from a difference value between the eighth sensing time (LS2'_OP) and the first sensing time (GS_OP) includes in particular: if a difference value between a tenth difference value and an eleventh difference value is less than a ninth threshold, determining the ventricle is captured, otherwise determining the ventricle is not captured;
where the tenth difference value is the difference value between the eighth sensing time (LS2'_OP) and the first sensing time (GS_OP), and the eleventh difference value is determined from a difference value between a ninth sensing time (LS2'_SUP) and a seventh sensing time (GS'_SUP); and the ninth sensing time (LS2'_SUP) is a sensing time of ventricular muscle activation corresponding to a pacing event in an in-vivo near-field myocardial electrogram acquired by the second electrode pair during the ventricle is captured by spacing within a set up period, and the seventh sensing time (GS'_SUP) is a sensing time of an R-wave in a far-field cardiac electrogram acquired during the ventricle is captured by pacing within a set up period.

Optionally, the step of determining whether the ventricle is captured from a difference value between the eighth sensing time (LS2'_OP) and the first sensing time (GS_OP) includes in particular: if a ratio of a tenth difference value to a twelfth difference value is greater than a tenth threshold, determining the ventricle is captured, otherwise determining the ventricle is not captured;
where the tenth difference value is the difference value between the eighth sensing time (LS2'_OP) and the first sensing time (GS_OP), and the twelfth difference value is determined from a difference value between a sixth sensing time (LS2_SUP) and a third sensing time (GS_SUP); and the sixth sensing time (LS2_SUP) is a sensing time corresponding to a sensed event in an in-vivo near-field myocardial electrogram acquired by the second electrode pair within a set up period, the third sensing time (GS_SUP) is a sensing time of an R-wave in a far-field cardiac electrogram acquired within a set up period, and the set up period corresponds to atrium originated ventricular activation.

Optionally, the control device is further configured to perform, during the operational period, the following step: determining the origin of ventricular activation of the heartbeat according to a difference value between or a ratio of a first specific difference value and a second specific difference value;
where the first specific difference value is the difference value between the first specific time and the second specific time, the second specific difference value is a difference value between a third specific time and a fourth specific time, and the third specific time and the fourth specific time are selected from the following times acquired within a set up period: sensing time (GS_SUP) of an R-wave in a far-field cardiac electrogram, sensing time (GS'_SUP) of an R-wave in a far-field cardiac electrogram acquired during the ventricle is captured by pacing, sensing time (LS2_SUP) corresponding to a sensed event in a second in-vivo near-field myocardial electrogram acquired by the second electrode pair, sensing time (LS2'_SUP) of ventricular muscle activation corresponding to a pacing event in a second in-vivo near-field myocardial electrogram acquired by the second electrode pair, sensing time (LS1_SUP) corresponding to a sensed event in a first in-vivo near-field myocardial electrogram acquired by the first electrode pair, and time (VP_SUP) when a pacing pulse is delivered through the first electrode pair during the ventricle is captured by pacing.

Optionally, the control device being further configured to perform, during the operational period, the following step includes in particular:
after a first preset number of heartbeats within the operational period, the control device being configured to perform the following step:
for a second preset number of heartbeats, if it is determined that there are a third preset number of heartbeats as paced heartbeats, determining that the ventricle has been captured, otherwise determining that the ventricle is being captured;
where the third preset number is less than or equal to the second preset number.

Optionally, the medical instrument further includes at least one ventricular electrode lead, the at least one ventricular electrode lead being further configured with the first electrode pair and/or the second electrode pair for sensing.

Optionally, the first sensed event and/or the second sensed event is ventricular activation, and the first electrode pair and/or the second electrode pair is an electrode pair for attachment to a ventricle or an epicardium of the ventricle.

On the basis of conforming to the common knowledge in the art, the above optional conditions may be combined arbitrarily to obtain preferred embodiments of the present application.

The following are the positive and improved effects of the present application. The medical instrument provided in the present application enables the classification of the origin of ventricular activation of the heartbeat for the operational period using only the temporal information of the ventricle, with no temporal information of the atrium and without waiting until a complete ventricular activation of the heart is completed and even with no use of morphological algorithms. The classification is simple and has no effect on the energy consumption required by the medical instrument.

The medical instrument provided in the present application can use only the temporal information of the ventricle to determine the ventricle is captured for the operational period, the determination is simple and has no effect on the energy consumption required by the medical instrument.

### BRIEF DESCRIPTION OF THE DRAWINGS

Fig. 1A shows a schematic representation of a far-field cardiac electrogram and an in-vivo near-field myocardial electrogram of an atrium originated ventricular activation.
Fig. 1B shows a schematic representation of a far-field cardiac electrogram and an in-vivo near-field myocardial electrogram of a ventricle originated ventricular activation.
Fig. 2A shows a schematic representation of a far-field cardiac electrogram, a first in-vivo near-field myocardial electrogram and a second in-vivo near-field myocardial electrogram of an atrium originated ventricular activation.
Fig. 2B shows a schematic representation of a far-field cardiac electrogram, a first in-vivo near-field myocardial electrogram and a second in-vivo near-field myocardial electrogram of a ventricle originated ventricular activation.
Fig. 3 shows a flowchart of a method for controlling a medical instrument according to an embodiment of the present application.
Fig. 4 shows a schematic representation of a control device connected to an electrode pair according to an embodiment of the present application.
Fig. 5 shows a flowchart of a method for controlling a medical instrument according to Embodiment 1 of the present application.
Fig. 6 shows a schematic representation of waveforms of a far-field cardiac electrogram and a near-field electrogram for a set up period and an operational period.
Fig. 7 shows a schematic representation of waveforms of a far-field cardiac electrogram and a near-field electrogram for an operational period.
Fig. 8 shows a flowchart of a method for controlling a medical instrument according to Embodiment 2 of the present application.
Fig. 9 shows a schematic representation of a far-field cardiac electrogram and an in-vivo near-field myocardial electrogram according to the Embodiment 2 of the present application.
Fig. 10 shows a flowchart of a method for controlling a medical instrument according to Embodiment 3 of the present application.
Fig. 11 shows a schematic representation of other waveforms of the far-field cardiac electrogram and the near-field electrogram for the set up period and the operational period.
Fig. 12 shows a flowchart of a method for controlling a medical instrument according to Embodiment 4 of the present application.
Fig. 13 shows a schematic representation of waveforms of a near-field electrogram for the set up period and the operational period.
Fig. 14 shows a schematic representation of other waveforms of the near-field electrogram for the set up period and the operational period.
Fig. 15 shows a flowchart of a method for controlling a medical instrument according to Embodiment 5 of the present application.
Fig. 16 shows a schematic representation of other waveforms of the far-field cardiac electrogram and the near-field electrogram for the set up period and the operational period.

### DETAILED DESCRIPTION OF THE PREFERRED EMBODIMENT

The present application is further illustrated by way of embodiment below, but is not thereby limited to the scope of the described embodiments.

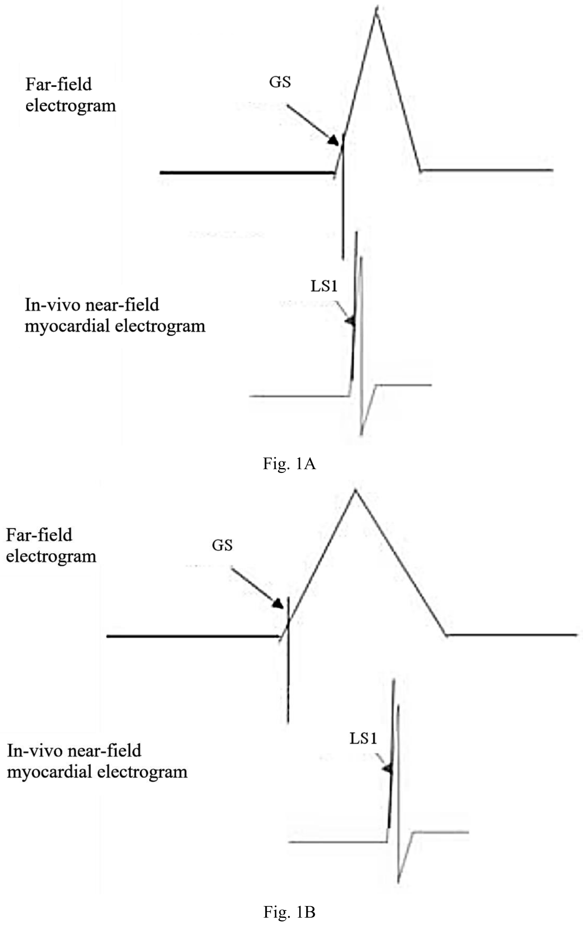

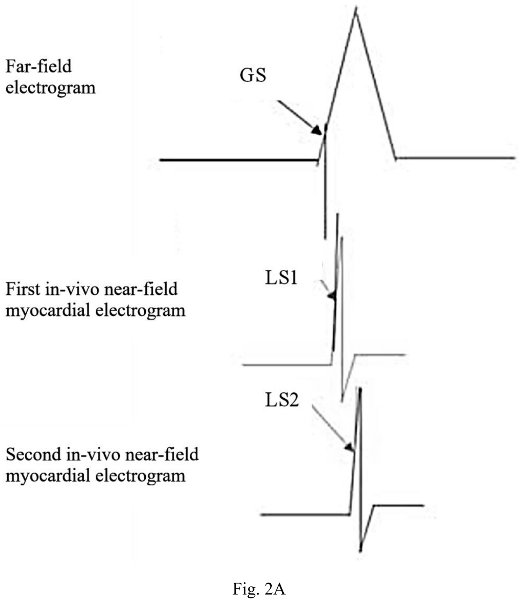

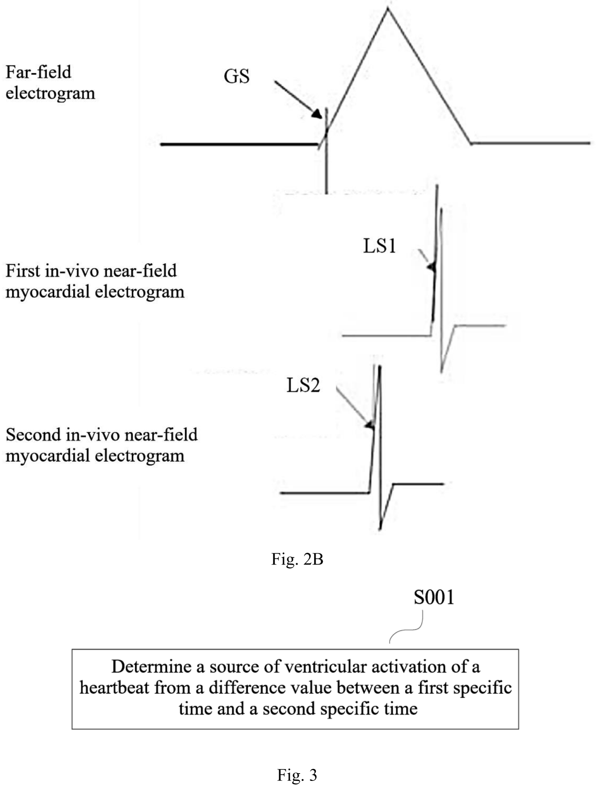

A far-field cardiac electrogram is used to sense the overall electrical activity of the heart, and an RTW (R-wave time window) corresponds to a time window of the overall ventricular activation seen in a far-field cardiac electrogram (FF-EGM). This means that all ventricular activation signals measured at various regional myocardia of the ventricle should fall within the RTW. It can be seen that a ventricular activation signal LS1 in an in-vivo near-field myocardial electrogram (NF-EGM) of an atrium originated ventricular activation of Fig. 1A falls within a RTW, a ventricular activation signal LS1 in an in-vivo near-field myocardial electrogram of a ventricle originated ventricular activation of Fig. 1B also falls within a RTW, both a ventricular activation signal LS1 in a first in-vivo near-field myocardial electrogram and a ventricular activation signal LS2 in a second in-vivo near-field myocardial electrogram of an atrium originated ventricular activation of Fig. 2A fall within a RTW, and both a ventricular activation signal LS1 in a first in-vivo near-field myocardial electrogram and a ventricular activation signal LS2 in a second in-vivo near-field myocardial electrogram of a ventricle originated ventricular activation of Fig. 2B also fall within a RTW.

It should be noted that the medical instrument according to the embodiments of the present application may particularly be an implantable medical instrument, such as an implantable single-chamber pacemaker, a dual-chamber pacemaker, a single-chamber ICD, a dual-chamber ICD, a cardiac resynchronization therapy defibrillator (CRT-D), a cardiac resynchronization therapy pacemaker (CRT-P), or CCM, or a temporary or long-term in vitro medical instrument having similar single and/or combined functions.

A set up period (SUP) mentioned in the embodiments of the present application may be a period during which the medical instrument does not actually operate. For example, during the process of a surgeon implanting a medical instrument for a patient, the set up period mentioned in the embodiments of the present application may also be a period during which the medical instrument actually operates. For example, the set up period is a period of time for which the patient is hospitalized, and within which a surface far-field cardiac electrogram or an in vivo far-field cardiac electrogram of the patient can be obtained by surface electrodes or temporary intracardiac electrode leads. For another example, the set up period is a period of time prior to an operational period, during which an actual surface far-field cardiac electrogram or in-vivo far-field cardiac electrogram is obtained using electrodes and associated devices. The operational period (OP) is a period during which the medical instrument actually operates. Parameters (such as sensing time) obtained within the set up period in the embodiments of the present application may be calculated values over a plurality of cardiac cycles under a certain relatively stable cardiac rhythm, such as a mean, a median, or another statistical value. Optionally, data within the set up period may also be updated and used within the operational period. Alternatively, preset data may be updated and used when the patient returns to the hospital for reexamination.

The in-vivo near-field myocardial electrogram mentioned in the embodiments of the present application is also referred to as a local electrogram (L-EGM) and sometimes also referred to as a near-field electrogram, and the far-field cardiac electrogram may also be referred to as a far-field electrogram (FF-EGM). The far-field R-wave mentioned in the embodiments of the present application is a short term for an R-wave in a far-field cardiac electrogram, which refers generally to an electrical signal recorded in a far field during the depolarization of a ventricular muscle and corresponds to an R-wave in an in vivo (e.g., intracardiac or epicardial) far-field myocardial electrogram (EGM) or to a QRS wave in an electrocardiogram (ECG) and also includes various forms of QRS waves.

A difference value between two difference values mentioned in the present application usually refers to an absolute value of the difference value between the two difference values, and a ratio between the two difference values usually refers to a ratio between the absolute values of the two difference values. In a specific example, a difference value between a first difference value and a second difference value refers to |first difference value - second difference value|, and a ratio between the first difference value and the second difference value refers to |first difference value|/|second difference value|.

A medical instrument according to the embodiments of the present application includes a control device. As shown in Fig. 3, the control device is configured to perform, during an operational period, the following step S001.

In step S001, the origin of ventricular activation of a heartbeat is determined from a difference value between a first specific time and a second specific time.

The first specific time and the second specific time are selected from the following times acquired within the operational period: sensing time (LS1_OP) corresponding to a sensed event in a first in-vivo near-field myocardial electrogram acquired on the basis of a first electrode pair, time when a pacing pulse is delivered through the first electrode pair (VP_OP), sensing time (GS_OP) of an R-wave in a far-field cardiac electrogram, sensing time (LS2_OP) corresponding to a second sensed event in a second in-vivo near-field myocardial electrogram acquired on the basis of a second electrode pair, and sensing time (LS2'_OP) of ventricular muscle activation corresponding to a pacing event in the second in-vivo near-field myocardial electrogram acquired by the second electrode pair.

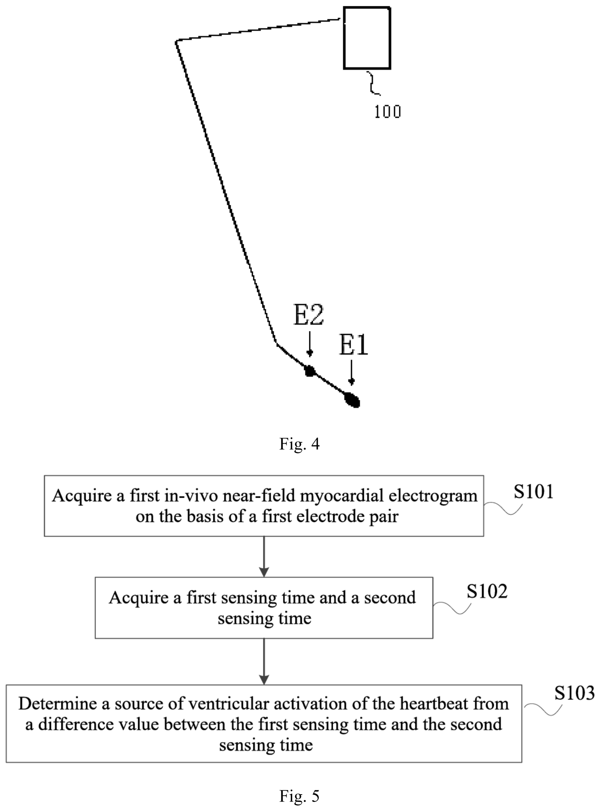

Fig. 4 shows a schematic representation of a control device connected to an electrode pair. As shown in Fig. 4, a control device 100 is connected to an electrode pair including an electrode E1 and an electrode E2. The electrode pair shown in Fig. 4 may be a first electrode pair or a second electrode pair.

In an optional implementation, the control device is further configured to perform, during an operational period, the following step: determining the origin of ventricular activation of the heartbeat according to a difference value between or a ratio of a first specific difference value and a second specific difference value.

The first specific difference value is the difference value between the first specific time and the second specific time, the second specific difference value is a difference value between a third specific time and a fourth specific time, and the third specific time and the fourth specific time are selected from the following times acquired within a set up period: sensing time (GS_SUP) of an R-wave in a far-field cardiac electrogram, sensing time (GS'_SUP) of an R-wave in a far-field cardiac electrogram acquired during the ventricle is captured by pacing, sensing time (LS2_SUP) corresponding to a sensed event in a second in-vivo near-field myocardial electrogram acquired by the second electrode pair, sensing time (LS2'_SUP) of ventricular muscle activation corresponding to a pacing event in a second in-vivo near-field myocardial electrogram acquired by the second electrode pair, sensing time (LS1_SUP) corresponding to a sensed event in a first in-vivo near-field myocardial electrogram acquired by the first electrode pair, and time (VP_SUP) when a pacing pulse is delivered through the first electrode pair during the ventricle is captured by pacing.

In an optional implementation, the medical instrument includes a control device and at least one ventricular electrode lead. The at least one ventricular electrode lead is configured with a first electrode pair and/or a second electrode pair for sensing. In a specific example, the medical instrument includes a control device and one ventricular electrode lead, and the ventricular electrode lead is configured with a first electrode pair and/or a second electrode pair for sensing. In another specific example, the medical instrument includes a control device and two ventricular electrode leads. Each of the ventricular electrode leads is configured with a first electrode pair and/or a second electrode pair for sensing. In another specific example, the medical instrument includes a control device and two ventricular electrode leads, and only one of the ventricular electrode leads is configured with a first electrode pair and/or a second electrode pair for sensing. In another specific example, the medical instrument includes two ventricular electrode leads. One of the ventricular electrode leads is configured with a first electrode pair for sensing and the other of the ventricular electrode leads is configured with a second electrode pair for sensing.

In another optional implementation, the medical instrument includes a control device for connection to at least one ventricular electrode lead. The at least one ventricular electrode lead is configured with a first electrode pair/or a second electrode pair for sensing. The first electrode pair/or the second electrode pair is an electrode pair for attachment to a ventricle or an epicardium of the ventricle. In a specific example, the control device is configured to be connected to one ventricular electrode lead, and the ventricular electrode lead is configured with a first electrode pair/or a second electrode pair for sensing. In another specific example, the control device is configured to be connected to two ventricular electrode leads, and each of the ventricular electrode leads is configured with a first electrode pair/or a second electrode pair for sensing. In another specific example, the control device is configured to be connected to two ventricular electrode leads, and only one of the ventricular electrode leads is configured with a first electrode pair/or a second electrode pair for sensing. In another specific example, the control device is configured to be connected to two ventricular electrode leads. One of the ventricular electrode leads is configured with a first electrode pair for sensing and the other of the ventricular electrode leads is configured with a second electrode pair for sensing.

In another optional implementation, the medical instrument includes a control device that is directly connected to the first electrode pair and/or the second electrode pair.

### Embodiment 1

Fig. 5 shows a schematic flowchart of a method for controlling a medical instrument according to this embodiment. The control method can be performed by a control device of the medical instrument during the operational period. The control device can be implemented in software and/or hardware. The control device may be a part of the medical instrument. The control device can be used in vitro or implanted in a human body.

As shown in Fig. 5, the method for controlling a medical instrument according to this embodiment may include the following steps S101-S103.

In step S101, a first in-vivo near-field myocardial electrogram is acquired on the basis of a first electrode pair.

In step S102, a first sensing time and a second sensing time are acquired. The first sensing time is the sensing time of the R-wave in a far-field cardiac electrogram, and the second sensing time is the sensing time corresponding to the sensed event in the first in-vivo near-field myocardial electrogram. The R-wave in the far-field cardiac electrogram and the sensed event in the first in-vivo near-field myocardial electrogram correspond to the same heartbeat. In this embodiment, the first sensing time is a first specific time, and the second sensing time is a second specific time.

In step S103, the origin of ventricular activation of the heartbeat is determined from a difference value between the first sensing time and the second sensing time. The origin of ventricular activation includes atrium originated ventricular activation and ventricle originated ventricular activation.

It is assumed that the first sensing time GS_OP and the second sensing time LS1_OP are acquired within the operational period. In this implementation, whether the origin of ventricular activation of the heartbeat for the operational period is atrium originated ventricular activation or ventricle originated ventricular activation is determined based on GS_OP-LS1_OP, that is, the origin of ventricular activation of the heartbeat for the operational period can be classified using only the temporal information of the ventricle, with no temporal information of the atrium and without waiting until a complete ventricular activation of the heart is completed and even with no use of morphological algorithms. The classification is simple and has no effect on the energy consumption required for the medical instrument.

Further, when it is determined that the origin of ventricular activation of the heartbeat for the operational period is atrium originated ventricular activation, the medical instrument can be controlled to provide cardiac therapy to the patient, such as the delivery of CCM pulses, which can improve the efficacy and patient safety.

In an optional implementation of step S103, if a difference value between a first difference value and a second difference value is greater than a first threshold, it is determined that the origin of ventricular activation of the heartbeat is ventricle originated ventricular activation, otherwise it is determined that the origin of ventricular activation of the heartbeat is atrium originated ventricular activation. The first difference value is a difference value between the first sensing time and the second sensing time, and the second difference value is determined from a difference value between a third sensing time and a fourth sensing time. The third sensing time is a sensing time of an R-wave in a far-field cardiac electrogram acquired within the set up period, and the fourth sensing time is a sensing time corresponding to a sensed event in an in-vivo near-field myocardial electrogram acquired by the first electrode pair within the set up period. The origin of ventricular activation of the heartbeat corresponding to the set up period is atrium originated ventricular activation.

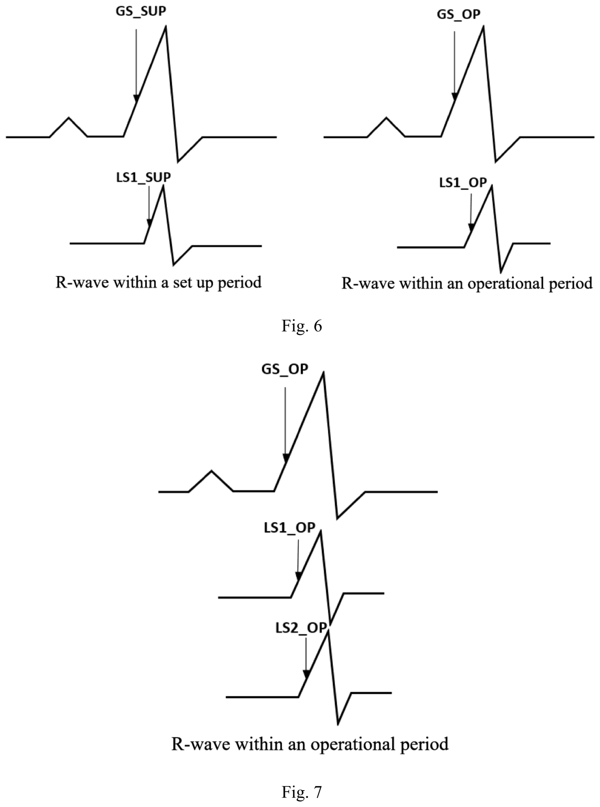

As shown in Fig. 6, it is assumed that the first sensing time GS_OP and the second sensing time LS1_OP are acquired within the operational period, the first difference value is GS_OP-LS1_OP, the third sensing time GS_SUP and the fourth sensing time LS1_SUP are acquired within the set up period, and the second difference value is GS_SUP-LS1_SUP. If the difference value between the first difference value and the second difference value is greater than the first threshold, that is, |(GS_OP-LS1_OP) - (GS_SUP-LS1_SUP)| is greater than the first threshold, it is determined that the origin of ventricular activation of the heartbeat is ventricle originated ventricular activation; and if the difference value between the first difference value and the second difference value is less than or equal to the first threshold, that is, |(GS_OP-LS1_OP) - (GS_SUP-LS1_SUP)| is less than or equal to the first threshold, it is determined that the origin of ventricular activation of the heartbeat is atrium originated ventricular activation. The first threshold may be set according to actual situations. For example, the first threshold may be selected from the range of values of 0-100 ms, and preferably may be set to 10 ms. The first threshold may also be obtained in other ways, for example, by being directly programmed by an external device (such as a programmer) by the surgeon inputting the first threshold to modify a preset value. In this implementation, the third sensing time is a third specific time, and the fourth sensing time is a fourth specific time.

In this implementation, the origin of ventricular activation of the heartbeat corresponding to the set up period is atrium originated ventricular activation, and if the difference value between the first difference value calculated for the operational period and the second difference value calculated for the set up period is greater than the first threshold, it is indicated that the first difference value calculated for the operational period is significantly different from the second difference value calculated for the set up period, and in this case, it is determined that the origin of ventricular activation of the heartbeat for the operational period is ventricle originated ventricular activation, otherwise it is determined that the origin of ventricular activation of the heartbeat for the operational period is atrium originated ventricular activation. Due to the specificity of an individual, by the method, the origin of ventricular activation can be accurately determined by comparing sensed data of the patient within the set up period with sensed data within the operational period, to provide a more accurate and specific determination result.

In a specific implementation, the second difference value GS_SUP-LS1_SUP calculated for the set up period may be a value calculated over a plurality of cardiac cycles, such as a mean, a median, a quartile, or another statistical value.

In another optional implementation of step S103, if the difference value between the first sensing time and the second sensing time is greater than a second threshold, it is determined that the origin of ventricular activation of the heartbeat is ventricle originated ventricular activation, otherwise it is determined that the origin of ventricular activation of the heartbeat is atrium originated ventricular activation.

It is assumed that the first sensing time GS_OP and the second sensing time LS1_OP are acquired within the operational period. If GS_OP-LS1_OP is greater than the second threshold, it is indicated that the difference value between the first sensing time and the second sensing time is large, and in this case, it is determined that the origin of ventricular activation of the heartbeat for the operational period is ventricle originated ventricular activation, otherwise it is determined that the origin of ventricular activation of the heartbeat for the operational period is atrium originated ventricular activation or another ventricular activation. The second threshold may be set according to actual situations. For example, the second threshold may be selected from the range of values of 0-150 ms, and preferably, for example, may be set to 100 ms. The second threshold may also be obtained in other ways, for example, by being directly programmed by an external device (such as a programmer) by the surgeon inputting the second threshold to modify a preset value.

In an optional implementation, the above control method further includes the following step: acquiring a second in-vivo near-field myocardial electrogram on the basis of a second electrode pair. In this implementation, the above step S103 includes in particular: determining the origin of ventricular activation of the heartbeat according to the difference value between the first sensing time and the second sensing time and a difference value between the first sensing time and a fifth sensing time. The fifth sensing time is the sensing time corresponding to the sensed event in the second in-vivo near-field myocardial electrogram, and the sensed event in the second in-vivo near-field myocardial electrogram and the R-wave in the far-field cardiac electrogram correspond to the same heartbeat.

As shown in Fig. 7, it is assumed that the first sensing time GS_OP, the second sensing time LS1_OP and the fifth sensing time LS2_OP are acquired within the operational period, the origin of ventricular activation of the heartbeat for the operational period can be determined from GS_OP-LS1_OP alone or in combination of GS_OP-LS1_OP and GS_OP-LS2_OP. In a specific example, if it is determined that the ventricular activation of the heartbeat for the operational period is ventricle originated ventricular activation from GS_OP-LS1_OP, it may be finally determined for the heartbeat that the origin of ventricular activation is ventricle originated ventricular activation. In another specific example, if it is determined that the ventricular activation of the heartbeat for the operational period is atrium originated ventricular activation from GS_OP-LS1_OP, the origin of ventricular activation of the heartbeat may also be finally further determined from GS_OP-LS2_OP. When the determination results of both are the same, the origin of ventricular activation of the heartbeat is finally determined. In this example, the origin of ventricular activation of the heartbeat for the operational period can be determined by two electrode pairs, such that a more accurate determination result can be obtained.

### Embodiment 2

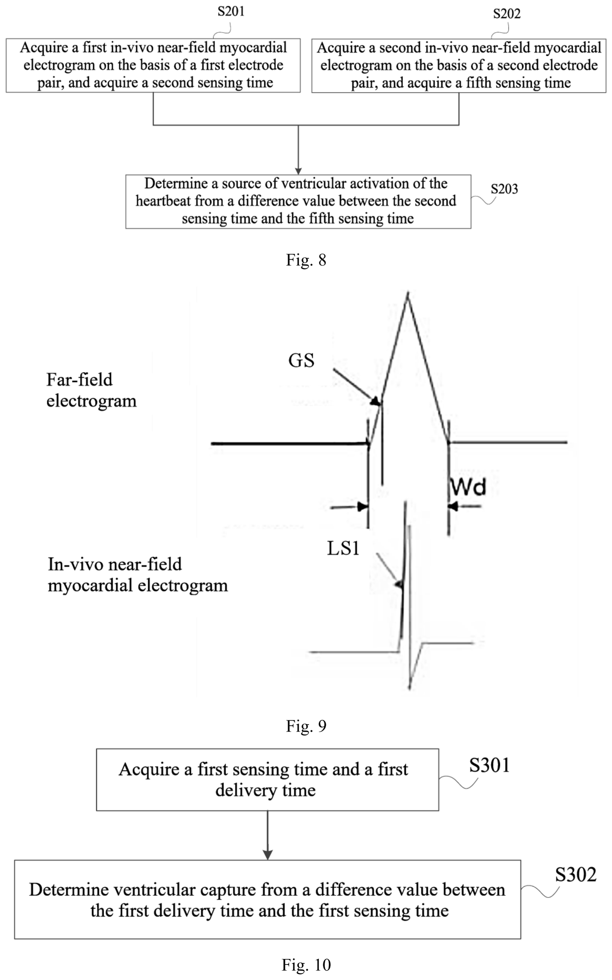

Fig. 8 shows a schematic flowchart of a method for controlling a medical instrument according to this embodiment. The control method can be performed by a control device of the medical instrument during the operational period. The control device can be implemented in software and/or hardware. The control device may be a part of the medical instrument. The control device can be used in vitro or implanted in a human body.

As shown in Fig. 8, the method for controlling a medical instrument according to this embodiment may include the following steps S201-S203.

In step S201, a first in-vivo near-field myocardial electrogram is acquired on the basis of a first electrode pair, and a second sensing time is acquired. The second sensing time is the sensing time corresponding to the sensed event in the first in-vivo near-field myocardial electrogram.

In step S202, a second in-vivo near-field myocardial electrogram is acquired on the basis of a second electrode pair, and a fifth sensing time is acquired. The fifth sensing time is the sensing time corresponding to a sensed event in the second in-vivo near-field myocardial electrogram, and the sensed event in the second in-vivo near-field myocardial electrogram and the sensed event in the first in-vivo near-field myocardial electrogram correspond to the same heartbeat.

In step S203, the origin of ventricular activation of the heartbeat is determined from a difference value between the second sensing time and the fifth sensing time. The origin of ventricular activation includes atrium originated ventricular activation and ventricle originated ventricular activation. In this embodiment, the second sensing time is a first specific time, and the fifth sensing time is a second specific time.

It is assumed that the second sensing time LS1_OP and the fifth sensing time LS2_OP are acquired within the operational period. In this implementation, whether the origin of ventricular activation of the heartbeat for the operational period is atrium originated ventricular activation or ventricle originated ventricular activation is determined based on LS1_OP-LS2_OP, that is, the origin of ventricular activation of the heartbeat for the operational period can be classified using only the temporal information of the ventricle, with no temporal information of the atrium, without waiting until a complete ventricular activation of the heart is completed, with no reference to a far-field cardiac electrogram and even with no use of morphological algorithms. The classification is simple and has no effect on the energy consumption required by the medical instrument.

Further, when it is determined that the origin of ventricular activation of the heartbeat for the operational period is atrium originated ventricular activation, the medical instrument can be controlled to provide cardiac therapy to the patient, such as the delivery of CCM pulses, which can improve the efficacy and patient safety.

In an optional implementation of step S203, if a difference value between a third difference value and a fourth difference value is greater than a third threshold, it is determined that the origin of ventricular activation of the heartbeat is ventricle originated ventricular activation, otherwise it is determined that the origin of ventricular activation of the heartbeat is atrium originated ventricular activation. The third difference value is a difference value between the second sensing time and the fifth sensing time, and the fourth difference value is determined from a difference value between a fourth sensing time and a sixth sensing time. The fourth sensing time is a sensing time corresponding to a sensed event in an in-vivo near-field myocardial electrogram acquired by the first electrode pair within the set up period, and the sixth sensing time is a sensing time corresponding to a sensed event in an in-vivo near-field myocardial electrogram acquired by the second electrode pair within the set up period. The origin of ventricular activation of the heartbeat corresponding to the set up period is atrium originated ventricular activation.

It is assumed that the second sensing time LS1_OP and the fifth sensing time LS2_OP are acquired within the operational period, the third difference value is LS1_OP-LS2_OP, the fourth sensing time LS1_SUP and the sixth sensing time LS2_SUP are acquired within the set up period, and the fourth difference value is LS1_SUP-LS2_SUP. If the difference value between the third difference value and the fourth difference value is greater than the third threshold, that is, |(LS1_OP-LS2_OP) - (LS1_SUP-LS2_SUP)| is greater than the third threshold, it is determined that the origin of ventricular activation of the heartbeat is ventricle originated ventricular activation; and if the difference value between the third difference value and the fourth difference value is less than or equal to the third threshold, that is, |(LS1_OP-LS2_OP) - (LS1_SUP-LS2_SUP)| is less than or equal to the third threshold, it is determined that the origin of ventricular activation of the heartbeat is atrium originated ventricular activation. The third threshold may be set according to actual situations, for example, may be set to 10 ms. The third threshold may also be obtained in other ways, for example, by being directly programmed by an external device (such as a programmer) by the surgeon inputting the third threshold to modify a preset value. In this implementation, the fourth sensing time is a third specific time, and the sixth sensing time is a fourth specific time.

In this implementation, the origin of ventricular activation of the heartbeat corresponding to the set up period is atrium originated ventricular activation, and if the difference value between the third difference value calculated for the operational period and the fourth difference value calculated for the set up period is greater than the third threshold, it is indicated that the third difference value calculated for the operational period is significantly different from the fourth difference value calculated for the set up period, and in this case, it is determined that the origin of ventricular activation of the heartbeat for the operational period is ventricle originated ventricular activation, otherwise it is determined that the origin of ventricular activation of the heartbeat for the operational period is atrium originated ventricular activation.

In another optional implementation of step S203, the origin of ventricular activation of the heartbeat may also be determined from a ratio of the third difference value to the fourth difference value, and particularly if the ratio of the third difference value to the fourth difference value is not within a preset range, it is indicated that the third difference value calculated for the operational period is significantly different from the fourth difference value calculated for the set up period, and in this case, it is determined that the origin of ventricular activation of the heartbeat for the operational period is ventricle originated ventricular activation, otherwise it is determined that the origin of ventricular activation of the heartbeat is atrium originated ventricular activation. The preset range may be set according to specific situations, for example, may be set to 0.95-1.05.

In a specific implementation, the fourth difference value LS1_SUP-LS2_SUP calculated for the set up period may be determined from difference values between the fourth sensing times and the sixth sensing times corresponding to a plurality of heartbeats, for example may be a mean, a median, a quartile, or another statistical value of the plurality of difference values.

In another optional implementation of step S203, if the difference value between the second sensing time and the fifth sensing time is greater than a fourth threshold, it is determined that the origin of ventricular activation of the heartbeat is ventricle originated ventricular activation, otherwise it is determined that the origin of ventricular activation of the heartbeat is atrium originated ventricular activation.

It is assumed that the second sensing time LS1_OP and the fifth sensing time LS2_OP are acquired within the operational period. If LS1_OP-LS2_OP is greater than the fourth threshold, it is indicated that the difference value between the second sensing time and the fifth sensing time is large, and in this case, it is determined that the origin of ventricular activation of the heartbeat for the operational period is ventricle originated ventricular activation, otherwise it is determined that the origin of ventricular activation of the heartbeat for the operational period is atrium originated ventricular activation or another ventricular activation. The fourth threshold may be set according to actual situations. For example, the fourth threshold may be selected from the range of values of 0-150 ms, and preferably may be set to 90 ms. The fourth threshold may also be obtained in other ways, for example, by being directly programmed by an external device (such as a programmer) by the surgeon inputting the fourth threshold to modify a preset value.

In an optional implementation, the above control method further includes the following step: determining a length of a R-wave sensing time window corresponding to the R-wave in the far-field cardiac electrogram acquired within the set up period from the origin of ventricular activation of the heartbeat.

The R-wave sensing time window, which may also be referred to as RTW, corresponds to a window of ventricular activation seen in the far-field cardiac electrogram, and features its start time and length. It can be seen that a ventricular activation signal LS1 of the in-vivo near-field myocardial electrogram of Fig. 9 falls within the RTW, and the RTW has a length of Wd.

In a specific example, if the origin of ventricular activation of the heartbeat for the operational period is atrium originated ventricular activation, the width of the R-wave in the far-field cardiac electrogram is normal and typically less than 120 ms. The length of the R-wave sensing time window can be set to 90-100 ms. In another specific example, if the origin of ventricular activation of the heartbeat for the operational period is ventricle originated ventricular activation, the R-wave in the far-field cardiac electrogram is wide and typically between 160-250 ms. The length of the R-wave sensing time window can be set to 200-250 ms.

### Embodiment 3

Fig. 10 shows a schematic flowchart of a method for controlling a medical instrument according to this embodiment. The control method can be performed by a control device of the medical instrument during the operational period. The control device can be implemented in software and/or hardware. The control device may be a part of the medical instrument. The control device can be used in vitro or implanted in a human body.

As shown in Fig. 10, the method for controlling a medical instrument according to this embodiment may include the following steps S301-S302.

In step S301, a first sensing time and a first delivery time are acquired. The first sensing time is the sensing time of the R-wave in the far-field cardiac electrogram, and the first delivery time is time when a pacing pulse is delivered through the first electrode pair.

In step S302, the ventricle is captured is determined from a difference value between the first delivery time and the first sensing time.

It is assumed that the first sensing time GS_OP and the first delivery time VP_OP are acquired within the operational period. In this implementation, the the ventricle is captured is determined for the operational period from VP_OP-GS_OP, that is, the the ventricle is captured can be determined for the operational period using only the temporal information of the ventricle. The determination is simple and has no effect on the energy consumption required by the medical instrument. If the determination result is the ventricle is captured, it may be determined that the origin of ventricular activation of the heartbeat is ventricular pacing, and if the determination result is the ventricle is not captured, it may be determined that the origin of ventricular activation of the heartbeat is another autonomous ventricular activation instead of spacing. The first sensing time is a second specific time, and the first delivery time is a first specific time.

In an optional implementation of step S302, if a difference value between a fifth difference value and a sixth difference value is less than a fifth threshold, the ventricle is captured is determined, otherwise the ventricle is not captured is determined. The fifth difference value is a difference value between the first delivery time and the first sensing time, and the sixth difference value is determined from a difference value between a second delivery time and a seventh sensing time. The seventh sensing time is a sensing time of an R-wave in a far-field cardiac electrogram acquired during the ventricle is captured by pacing within a set up period, and the second delivery time is time when a pacing pulse is delivered through the first electrode pair during the ventricle is captured by pacing within the set up period.

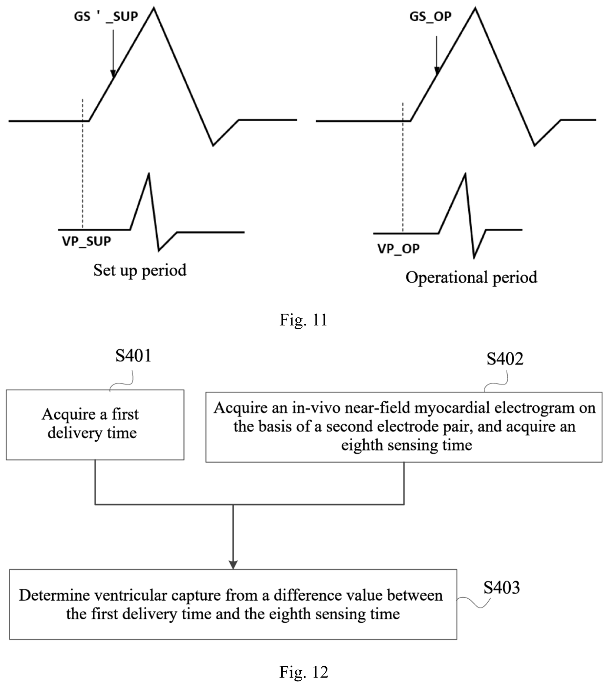

As shown in Fig. 11, it is assumed that the first sensing time GS_OP and the first delivery time VP_OP are acquired within the operational period, the fifth difference value is VP_OP-GS_OP, the seventh sensing time GS'_SUP and the second delivery time VP_SUP are acquired within the set up period, and the sixth difference value is VP_SUP-GS_SUP. If the difference value between the fifth difference value and the sixth difference value is less than the fifth threshold, that is, |(VP_OP-GS_OP) - (VP_SUP-GS'_SUP)| is less than the fifth threshold, the ventricle is captured is determined; and if the difference value between the fifth difference value and the sixth difference value is greater than or equal to the fifth threshold, that is, |(VP_OP-GS_OP) - (VP_SUP-GS'_SUP)| is greater than or equal to the fifth threshold, the ventricle is not captured is determined. The fifth threshold may be set according to actual situations, for example, may be set to 5 ms. The fifth threshold may also be obtained in other ways, for example, by being directly programmed by an external device (such as a programmer) by the surgeon inputting the fifth threshold to modify a preset value. In this implementation, the seventh sensing time is a fourth specific time, and the second delivery time is a third specific time.

In this implementation, the set up period corresponds to pacing and the ventricle is captured, and if the difference value between the fifth difference value calculated for the operational period and the sixth difference value calculated for the set up period is less than the fifth threshold, it is indicated that the fifth difference value calculated for the operational period is approximate to the sixth difference value calculated for the set up period, and in this case, the ventricle is captured is determined for the operational period, and the origin of ventricular activation of the heartbeat is ventricular pacing, otherwise the ventricle is not captured is determined for the operational period.

In a specific implementation, the sixth difference value calculated for the set up period may be determined from difference values between the second delivery times and the seventh sensing times corresponding to a plurality of heartbeats, for example may be a mean, a median, a quartile, or another statistical value of the plurality of difference values.

In another optional implementation of step S302, if the difference value between the first delivery time and the first sensing time is a positive number and is less than a sixth threshold, the ventricle is captured is determined, otherwise the ventricle is not captured is determined.

It is assumed that the first sensing time GS_OP and the first delivery time VP_OP are acquired within the operational period. If VP_OP-GS_OP is a positive number and is less than the sixth threshold, it is indicated that the first sensing time occurs after the first delivery time and that the difference value between the first sensing time and the first delivery time is small, and in this case, it is determined that the origin of ventricular activation for the operational period is ventricular pacing, i.e., the ventricle is captured, otherwise the ventricle is not captured is determined. The sixth threshold may be set according to actual situations, for example may be selected from the range of values of 0-100 ms, and preferably may be set to 40 ms. The sixth threshold may be obtained in other ways, for example by the sixth difference value, or by being directly programmed by an external device (such as a programmer) by the surgeon inputting the sixth threshold to modify a preset value.

### Embodiment 4

Fig. 12 shows a schematic flowchart of a method for controlling a medical instrument according to this embodiment. The control method can be performed by a control device of the medical instrument during the operational period. The control device can be implemented in software and/or hardware. The control device may be a part of the medical instrument. The control device can be used in vitro or implanted in a human body.

As shown in Fig. 12, the method for controlling a medical instrument according to this embodiment may include the following steps S401-S403.

In step S401, a first delivery time is acquired. The first delivery time is time when a pacing pulse is delivered through the first electrode pair.

In step S402, a second in-vivo near-field myocardial electrogram is acquired on the basis of a second electrode pair, and an eighth sensing time is acquired. The eighth sensing time is a sensing time of ventricular muscle activation corresponding to a pacing event in the second in-vivo near-field myocardial electrogram.

In step S403, the ventricle is captured is determined from a difference value between the first delivery time and the eighth sensing time. In this embodiment, the first delivery time is a first specific time, and the eighth sensing time is a second specific time.

It is assumed that the first delivery time VP_OP and the eighth sensing time LS2'_OP are acquired within the operational period. In this implementation, the the ventricle is captured is determined for the operational period from VP_OP-LS2'_OP, that is, the the ventricle is captured can be determined for the operational period using only the temporal information of the ventricle with no reference to a far-field cardiac electrogram. The determination is simple and has no effect on the energy consumption of the medical instrument.

In an optional implementation of step S403, if a ratio of a seventh difference value to an eighth difference value is greater than a seventh threshold, the ventricle is captured is determined, otherwise the ventricle is not captured is determined. The seventh difference value is a difference value between the first delivery time and the eighth sensing time, and the eighth difference value is determined from a difference value between a fourth sensing time and a sixth sensing time. The sixth sensing time is a sensing time corresponding to a sensed event in an in-vivo near-field myocardial electrogram acquired by the second electrode pair within the set up period, and the fourth sensing time is a sensing time corresponding to a sensed event in an in-vivo near-field myocardial electrogram acquired by the first electrode pair within the set up period. The origin of ventricular activation of the heartbeat corresponding to the set up period is atrium originated ventricular activation.

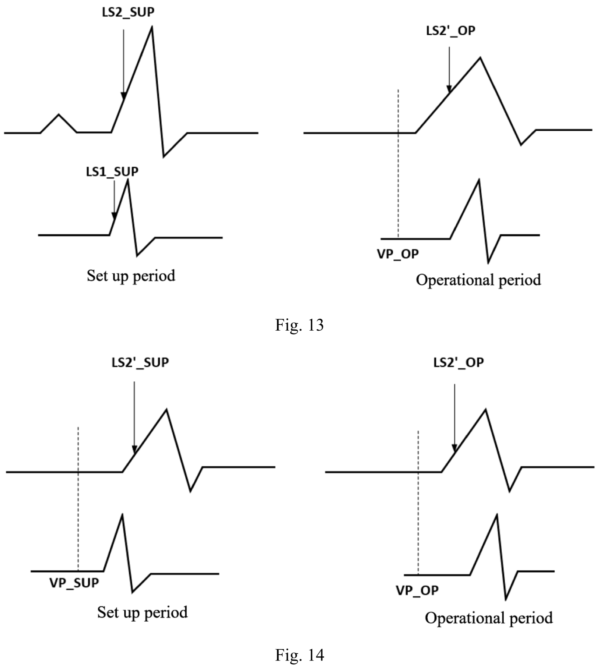

As shown in Fig. 13, it is assumed that the first delivery time VP_OP and the eighth sensing time LS2'_OP are acquired within the operational period, the seventh difference value is VP_OP-LS2'_OP, the sixth sensing time LS2_SUP and the fourth sensing time LS1_SUP are acquired within the set up period, and the eighth difference value is LS1_SUP-LS2_SUP. If the ratio of the seventh difference value to the eighth difference value is greater than the seventh threshold, that is, |VP_OP-LS2'_OP|/|LS1_SUP-LS2_SUP| is greater than the seventh threshold, the ventricle is captured is determined; and if the ratio of the seventh difference value to the eighth difference value is less than or equal to the seventh threshold, that is, |VP_OP-LS2'_OP|/|LS1_SUP-LS2_SUP| is less than or equal to the seventh threshold, the ventricle is not captured is determined. The seventh threshold may be set according to actual situations, for example may be selected from the range of values of 1.1-2.0, and preferably may be set to 1.3. The seventh threshold may be obtained in other ways, for example by acquiring the ratio of the eighth difference value between a ventricular activity originating from ventricular pacing and ventricular activity originating from the atrium within the set up period, or by being directly programmed by an external device (such as a programmer) by the surgeon inputting the seventh threshold to modify a preset value. In this implementation, the sixth sensing time is a third specific time, and the fourth sensing time is a fourth specific time.

In this implementation, the origin of ventricular activation of the heartbeat corresponding to the set up period is atrium originated ventricular activation, and if the difference value between the seventh difference value calculated for the operational period and the eighth difference value calculated for the set up period is greater than the seventh threshold, it is indicated that the seventh difference value calculated for the operational period is significantly different from the eighth difference value calculated for the set up period, and in this case, the ventricle is captured is determined for the operational period, otherwise the ventricle is not captured is determined for the operational period.

In another optional implementation of step S403, it is also possible to determine the ventricle is captured from the difference value between the seventh difference value and the eighth difference value, and particularly if the difference value between the seventh difference value and the eighth difference value is greater than a certain threshold, it is indicated that the seventh difference value calculated for the operational period is significantly different from the eighth difference value calculated for the set up period, and in this case, the ventricle is captured is determined for the operational period, otherwise the ventricle is not captured is determined for the operational period.

In another optional implementation of step S403, if a difference value between the seventh difference value and a ninth difference value is less than an eighth threshold, the ventricle is captured is determined, otherwise the ventricle is not captured is determined. The seventh difference value is a difference value between the first delivery time and the eighth sensing time, and the ninth difference value is determined from a difference value between a second delivery time and a ninth sensing time. The ninth sensing time is a sensing time of ventricular muscle activation corresponding to a pacing event in a second in-vivo near-field myocardial electrogram acquired by the second electrode pair during the ventricle is captured by spacing within the set up period, and the second delivery time is time when a pacing pulse is delivered through the first electrode pair during the ventricle is captured by pacing within the set up period. In this implementation, the ninth sensing time is a third specific time, and the second delivery time is a fourth specific time.

As shown in Fig. 14, it is assumed that the first delivery time VP_OP and the eighth sensing time LS2'_OP are acquired within the operational period, the seventh difference value is VP_OP-LS2'_OP, the ninth sensing time LS2'_SUP and the second delivery time VP_SUP are acquired within the set up period, and the ninth difference value is VP_SUP- LS2'_SUP. If the difference value between the seventh difference value and the ninth difference value is less than the eighth threshold, that is, |(VP_OP-LS2'_OP) - (VP_SUP-LS2'_SUP)| is less than the eighth threshold, the ventricle is captured is determined; and if the difference value between the seventh difference value and the ninth difference value is greater than or equal to the eighth threshold, that is, |(VP_OP-LS2'_OP) - (VP_SUP-LS2'_SUP)| is greater than or equal to the eighth threshold, the ventricle is not captured is determined. The eighth threshold may be set according to actual situations, for example, may be set to 5 ms. The eighth threshold may also be obtained in other ways, for example, by being directly programmed by an external device (such as a programmer) by the surgeon inputting the eighth threshold to modify a preset value.

In this implementation, the set up period corresponds to pacing and the ventricle is captured, and if the difference value between the seventh difference value calculated for the operational period and the ninth difference value calculated for the set up period is less than the eighth threshold, it is indicated that the seventh difference value calculated for the operational period is approximate to the ninth difference value calculated for the set up period, and in this case, the ventricle is captured is determined for the operational period, otherwise the ventricle is not captured is determined for the operational period.

### Embodiment 5

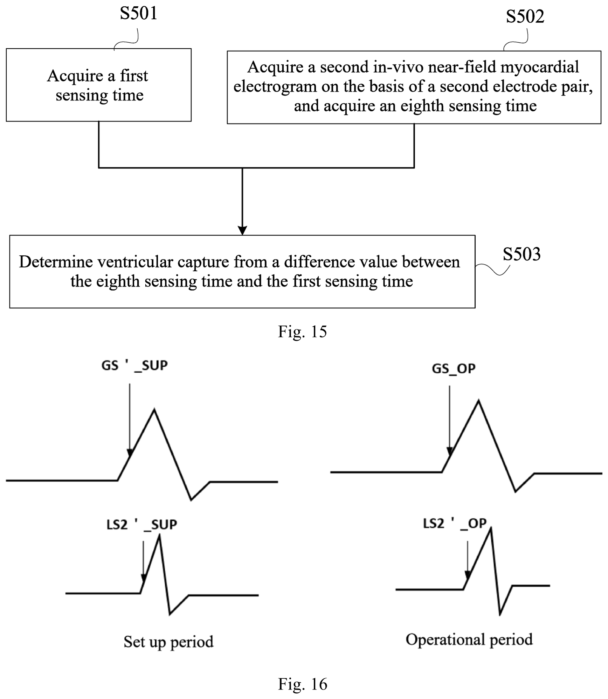

Fig. 15 shows a schematic flowchart of a method for controlling a medical instrument according to this embodiment. The control method can be performed by a control device of the medical instrument during the operational period. The control device can be implemented in software and/or hardware. The control device may be a part of the medical instrument. The control device can be used in vitro or implanted in a human body.

As shown in Fig. 15, the method for controlling a medical instrument according to this embodiment may include the following steps S501-S503.

In step S501, a first sensing time is acquired. The first sensing time is the sensing time of the R-wave in a far-field cardiac electrogram.

In step S502, a second in-vivo near-field myocardial electrogram is acquired on the basis of a second electrode pair, and an eighth sensing time is acquired. The eighth sensing time is a sensing time of ventricular muscle activation corresponding to a pacing event in the second in-vivo near-field myocardial electrogram.

In step S503, the ventricle is captured is determined from a difference value between the eighth sensing time and the first sensing time.

It is assumed that the first sensing time GS_OP and the eighth sensing time LS2'_OP are acquired within the operational period. In this implementation, the ventricle is captured is determined for the operational period from LS2'_OP-GS_OP, that is, the ventricle is captured can be determined for the operational period using only the temporal information of the ventricle and with only one non-spacing electrode pair. The determination is simple and has no effect on the energy consumption of the medical instrument. In this case, the first sensing time is a first specific time, and the eighth sensing time is a second specific time.

In an optional implementation of step S503, if a difference value between a tenth difference value and an eleventh difference value is less than a ninth threshold, the ventricle is captured is determined, otherwise the ventricle is not captured is determined. The tenth difference value is the difference value between the eighth sensing time and the first sensing time, and the eleventh difference value is determined from a difference value between a ninth sensing time and a seventh sensing time. The ninth sensing time is a sensing time of ventricular muscle activation corresponding to a pacing event in an in-vivo near-field myocardial electrogram acquired by the second electrode pair during the ventricle is captured by spacing within a set up period, and the seventh sensing time is a sensing time of an R-wave in a far-field cardiac electrogram acquired during the ventricle is captured by pacing within a set up period.

As shown in Fig. 16, it is assumed that the first sensing time GS_OP and the eighth sensing time LS2'_OP are acquired within the operational period, the tenth difference value is LS2'_OP-GS_OP, the ninth sensing time LS2'_SUP and the seventh sensing time GS'_SUP are acquired within the set up period, and the eleventh difference value is LS2'_SUP-GS'_SUP. If the difference value between the tenth difference value and the eleventh difference value is less than the ninth threshold, that is, |(LS2'_OP-GS_OP) - (LS2'_SUP-GS'_SUP)| is less than the ninth threshold, the ventricle is captured is determined; and if the difference value between the tenth difference value and the eleventh difference value is greater than or equal to the ninth threshold, that is, |(LS2'_OP-GS_OP) - (LS2'_SUP-GS'_SUP)| is greater than or equal to the ninth threshold, the ventricle is not captured is determined. The ninth threshold may be set according to actual situations, for example, may be set to 5 ms. The ninth threshold may also be obtained in other ways, for example, by being directly programmed by an external device (such as a programmer) by the surgeon inputting the ninth threshold to modify a preset value.

In this implementation, the set up period corresponds to pacing and the ventricle is captured, and if the difference value between the tenth difference value calculated for the operational period and the eleventh difference value calculated for the set up period is less than the ninth threshold, it is indicated that the tenth difference value calculated for the operational period is approximate to the eleventh difference value calculated for the set up period, and in this case, the ventricle is captured is determined for the operational period, otherwise the ventricle is not captured is determined for the operational period.

In another optional implementation of step S503, if a ratio of the tenth difference value to a twelfth difference value is greater than a tenth threshold, the ventricle is captured is determined, otherwise the ventricle is not captured is determined. The tenth difference value is a difference value between the eighth sensing time and the first sensing time, and the twelfth difference value is determined from a difference value between a sixth sensing time and a third sensing time. The sixth sensing time is a sensing time corresponding to a sensed event in an in-vivo near-field myocardial electrogram acquired by the second electrode pair within a set up period, and the third sensing time is a sensing time of an R-wave in a far-field cardiac electrogram acquired within a set up period. The set up period corresponds to atrium originated ventricular activation.

It is assumed that the first sensing time GS_OP and the eighth sensing time LS2'_OP are acquired within the operational period, the tenth difference value is LS2'_OP-GS_OP, the sixth sensing time LS2_SUP and the third sensing time GS_SUP are acquired within the set up period, and the twelfth difference value is LS2_SUP-GS_SUP. If the ratio of the tenth difference value to the twelfth difference value is greater than the tenth threshold, that is, |LS2'_OP-GS_OP|/|LS2_SUP-GS_SUP| is greater than the tenth threshold, the ventricle is captured is determined; and if the difference value between the tenth difference value and the twelfth difference value is less than or equal to the tenth threshold, that is, |LS2'_OP-GS_OP|/|LS2_SUP-GS_SUP| is less than or equal to the tenth threshold, the ventricle is not captured is determined. The tenth threshold may be set according to actual situations, for example may be set to 1.3. The tenth threshold may be obtained in other ways, for example by acquiring the ratio of the eighth difference value between a ventricular activity originating from ventricular pacing and ventricular activity originating from the atrium within the set up period, or by being directly programmed by an external device (such as a programmer) by the surgeon inputting the tenth threshold to modify a preset value.

In this implementation, the origin of ventricular activation of the heartbeat corresponding to the set up period is atrium originated ventricular activation, and if the ratio of the tenth difference value calculated for the operational period to the twelfth difference value calculated for the set up period is greater than the tenth threshold, it is indicated that the tenth difference value calculated for the operational period is significantly different from the twelfth difference value calculated for the set up period, and in this case, the ventricle is captured is determined for the operational period, otherwise the ventricle is not captured is determined for the operational period.

In another optional implementation of step S503, it is also possible to determine the ventricle is captured from the difference value between the tenth difference value and the twelfth difference value, and particularly if the difference value between the tenth difference value and the twelfth difference value is greater than an eleventh threshold, it is indicated that the tenth difference value calculated for the operational period is significantly different from the twelfth difference value calculated for the set up period, and in this case, the ventricle is captured is determined for the operational period, otherwise the ventricle is not captured is determined for the operational period.

The following implementations may also be incorporated in Embodiments 3-5, and the control device is configured to perform the above control method after a first preset number of heartbeats within the operational period. The first preset number can be set according to actual situations, for example, may be set to 5. In this implementation, the detection of the ventricle is captured being performed after the first preset number of heartbeats within the operational period allows to improve the accuracy of the detection.

In an optional implementation, for a second preset number of heartbeats, if it is determined that there are a third preset number of heartbeats as paced heartbeats, that is, heartbeats caused by pacing, it is determined that the ventricle has been captured, otherwise it is determined that the ventricle is being captured or has not yet been captured. It should be noted that the third preset number of heartbeats capturing the ventricle can be continuous or discontinuous.

The third preset number is less than or equal to the second preset number, and may be set according to actual situations. In a specific example, the second preset number is 12, and the third preset number is 9. In order to eliminate false positives or to minimize the false positives, the third preset number may be set to be equal to the second preset number.

The control device in the embodiments of the present application may be implemented in the form of software called by a processor. For example, the control device includes a processor and a memory, where the processor is connected to the memory, instructions and parameters are stored in the memory, including obtaining the parameters or preset parameters or a range of parameter within the set up period, and the processor calls the instructions and the parameters stored in the memory to implement the functions of a control unit. The processor is, for example, a general-purpose processor, such as a central processing unit, the memory may be integrated with or separate from the processor, for example an on-chip memory or an off-chip memory storing a computer program executable by the at least one processor. The computer program, when being executed by the at least one processor, causes the at least one processor to perform the control method according to this embodiment. Alternatively, the control unit may be implemented in the form of a hardware circuit, the function of which may be implemented by means of the design of the hardware circuit. The hardware circuit may be understood as one or more processors. For example, in an implementation, the hardware circuit is an application specific integrated circuit, and logical relationships between elements of the circuit are designed to implement the function of the above control unit. In the embodiments of the present application, the processor is a circuit with signal processing capability, and in an implementation, the processor may be an instruction-readable and executable circuit, for example a CPU, a microprocessor, or a digital signal processor, etc. In another implementation, the processor may implement a certain function by means of the logical relationship of the hardware circuit which is fixed or reconfigurable. For example, the processor is a hardware circuit implemented by an ASIC or PLD, such as field programmable gate array.

Although the specific implementations of the present application are described above, it should be appreciated by those skilled in the art that these are merely illustrative and that the scope of protection of the present application is defined by the appended claims. Various changes or modifications to these implementations may be made by those skilled in the art without departing from the principle and spirit of the present application, and these changes or modifications fall within the scope of the present application.

## Claims

1. A medical instrument, comprising a control device configured to perform, during an operational period, the following step:
determining an origin of ventricular activation of a heartbeat according to a difference value between a first specific time and a second specific time;
wherein the first specific time and the second specific time are selected from the following times acquired within the operational period: a sensing time (LS1_OP) corresponding to a first sensed event in a first in-vivo near-field myocardial electrogram acquired by a first electrode pair, a delivery time (VP_OP) of a pacing pulse by the first electrode pair, a sensing time (GS_OP) of an R-wave in a far-field cardiac electrogram, a sensing time (LS2_OP) corresponding to a second sensed event in a second in-vivo near-field myocardial electrogram acquired by a second electrode pair, and a sensing time (LS2'_OP) of ventricular muscle activation corresponding to a pacing event in the second in-vivo near-field myocardial electrogram acquired by the second electrode pair.

2. The medical instrument of claim 1, wherein the control device is further configured to perform, during the operational period, the following steps:
acquiring the first in-vivo near-field myocardial electrogram by the first electrode pair; and
acquiring a first sensing time (GS_OP) and a second sensing time (LS1_OP); wherein the first sensing time is the first specific time, and the first sensing time is the sensing time (GS_OP) of the R-wave in the far-field cardiac electrogram; the second sensing time is the second specific time, and the second sensing time is the sensing time (LS1_OP) corresponding to the sensed event in the first in-vivo near-field myocardial electrogram; and the R-wave in the far-field cardiac electrogram and the sensed event in the first in-vivo near-field myocardial electrogram correspond to the same heartbeat; and
the step of determining the origin of ventricular activation of the heartbeat according to a difference value between a first specific time and a second specific time comprises:
determining the origin of ventricular activation of the heartbeat according to a difference value between the first sensing time (GS_OP) and the second sensing time (LS1_OP); wherein the origin of ventricular activation comprises atrium originated ventricular activation and ventricle originated ventricular activation.

3. The medical instrument of claim 2, wherein the step of determining the origin of ventricular activation of the heartbeat according to a difference value between the first sensing time (GS_OP) and the second sensing time (LS1_OP) comprises:
if a difference value between a first difference value and a second difference value is greater than a first threshold, determining that the origin of ventricular activation of the heartbeat is ventricle originated ventricular activation, otherwise determining that the origin of ventricular activation of the heartbeat is atrium originated ventricular activation;
wherein the first difference value is the difference value between the first sensing time (GS_OP) and the second sensing time (LS1_OP), and the second difference value is a difference value between a third sensing time (GS_SUP) and a fourth sensing time (LS1_SUP); the third sensing time (GS_SUP) is a sensing time of an R-wave in a far-field cardiac electrogram acquired within a set up period, and the fourth sensing time (LS1_SUP) is a sensing time corresponding to a sensed event in an in-vivo near-field myocardial electrogram acquired by the first electrode pair within the set up period; and the origin of ventricular activation of the heartbeat corresponding to the set up period is atrium originated ventricular activation.

4. The medical instrument of claim 3, wherein the second difference value is determined according to difference values between the third sensing times (GS_SUP) and the fourth sensing times (LS1_SUP) corresponding to a plurality of heartbeats.

5. The medical instrument of claim 2, wherein the step of determining the origin of ventricular activation of the heartbeat according to the difference value between the first sensing time and the second sensing time comprises:
if the difference value between the first sensing time (GS_OP) and the second sensing time (LS1_OP) is greater than a second threshold, determining that the origin of ventricular activation of the heartbeat is ventricle originated ventricular activation, otherwise determining that the origin of ventricular activation of the heartbeat is another originated ventricular activation.

6. The medical instrument of claim 2, wherein the control device is further configured to perform, during the operational period, the following step:
acquiring the second in-vivo near-field myocardial electrogram by the second electrode pair; and
the step of determining the origin of ventricular activation of the heartbeat according to the difference value between the first sensing time (GS_OP) and the second sensing time (LS1_OP) comprises:
determining the origin of ventricular activation of the heartbeat according to the difference value between the first sensing time (GS_OP) and the second sensing time (LS1_OP) and a difference value between the first sensing time (GS_OP) and a fifth sensing time (LS2_OP);
wherein the fifth sensing time (LS2_OP) is the sensing time corresponding to the sensed event in the second in-vivo near-field myocardial electrogram, and the sensed event in the second in-vivo near-field myocardial electrogram and the R-wave in the far-field cardiac electrogram correspond to the same heartbeat.

7. The medical instrument of claim 1, wherein the control device is further configured to perform, during the operational period, the following steps:
acquiring a first in-vivo near-field myocardial electrogram by the first electrode pair and acquiring a second sensing time (LS1_OP); wherein the second sensing time is the first specific time, and the second sensing time is a sensing time corresponding to a sensed event in the first in-vivo near-field myocardial electrogram; and
acquiring a second in-vivo near-field myocardial electrogram by the second electrode pair and acquiring a fifth sensing time (LS2_OP); wherein the fifth sensing time is the second specific time, the fifth sensing time is a sensing time corresponding to a sensed event in the second in-vivo near-field myocardial electrogram, and the sensed event in the second in-vivo near-field myocardial electrogram and the sensed event in the first in-vivo near-field myocardial electrogram correspond to the same heartbeat; and
the step of determining the origin of ventricular activation of the heartbeat according to the difference value between the first specific time and the second specific time comprises:
determining the origin of ventricular activation of the heartbeat according to a difference value between the second sensing time (LS1_OP) and the fifth sensing time (LS2_OP); wherein the origin of ventricular activation comprises atrium originated ventricular activation and ventricle originated ventricular activation.

8. The medical instrument of claim 7, wherein the step of determining the origin of ventricular activation of the heartbeat according to a difference value between the second sensing time and the fifth sensing time comprises:
if a difference value between a third difference value and a fourth difference value is greater than a third threshold, determining that the origin of ventricular activation of the heartbeat is ventricle originated ventricular activation, otherwise determining that the origin of ventricular activation of the heartbeat is atrium originated ventricular activation;
wherein the third difference value is the difference value between the second sensing time (LS1_OP) and the fifth sensing time (LS2_OP), and the fourth difference value is determined from a difference value between a fourth sensing time (LS1_SUP) and a sixth sensing time (LS2_SUP); the fourth sensing time (LS1_SUP) is a sensing time corresponding to a sensed event in an in-vivo near-field myocardial electrogram acquired by the first electrode pair within a set up period, and the sixth sensing time is a sensing time (LS2_SUP) corresponding to a sensed event in an in-vivo near-field myocardial electrogram acquired by the second electrode pair within the set up period; and the origin of ventricular activation of the heartbeat corresponding to the set up period is atrium originated ventricular activation.

9. The medical instrument of claim 8, wherein the fourth difference value is determined from difference values between the fourth sensing times (LS1_SUP) and the sixth sensing times (LS2_SUP) corresponding to a plurality of heartbeats.

10. The medical instrument of claim 7, wherein the step of determining the origin of ventricular activation of the heartbeat according to a difference value between the second sensing time (LS1_OP) and the fifth sensing time (LS2_OP) comprises:
if the difference value between the second sensing time (LS1_OP) and the fifth sensing time (LS2_OP) is greater than a fourth threshold, determining that the origin of ventricular activation of the heartbeat is ventricle originated ventricular activation, otherwise determining that the origin of ventricular activation of the heartbeat is another originated ventricular activation.

11. The medical instrument of any one of claims 1-10, wherein the control device is further configured to perform, during the operational period, the following step:
determining a length of a R-wave sensing time window corresponding to the R-wave in the far-field cardiac electrogram acquired within the set up period according to the origin of ventricular activation of the heartbeat.

12. The medical instrument of claim 1, wherein the control device is further configured to perform, during the operational period, the following step:
acquiring a first sensing time (GS_OP) and a first delivery time (VP_OP); wherein the first sensing time (GS_OP) is the second specific time, the first sensing time is the sensing time of the R-wave in the far-field cardiac electrogram, the first delivery time (VP_OP) is the first specific time, and the first delivery time is time when a pacing pulse is delivered by the first electrode pair; and
the step of determining the source of the ventricular activation of the heartbeat according to the difference value between the first specific time and the second specific time comprises:
determining whether a ventricle is captured according to a difference value between the first delivery time (VP_OP) and the first sensing time (GS_OP).

13. The medical instrument of claim 12, wherein the step of determining whether the ventricle is captured according to a difference value between the first delivery time and the first sensing time comprises: if a difference value between a fifth difference value and a sixth difference value is less than a fifth threshold, determining the ventricle is captured, otherwise determining the ventricle is not captured;
wherein the fifth difference value is the difference value between the first delivery time (VP_OP) and the first sensing time (GS_OP), and the sixth difference value is determined according to a difference value between a second delivery time (VP_SUP) and a seventh sensing time (GS'_SUP); and the seventh sensing time is a sensing time (GS'_SUP) of an R-wave in a far-field cardiac electrogram acquired when the ventricle is captured by pacing within a set up period, and the second delivery time is time (VP_SUP) when a pacing pulse is delivered by the first electrode pair when the ventricle is captured by pacing within the set up period.

14. The medical instrument of claim 13, wherein the sixth difference value is determined according to difference values between the second delivery times (VP_SUP) and the seventh sensing times (GS'_SUP) corresponding to a plurality of heartbeats.

15. The medical instrument of claim 12, wherein the step of determining whether the ventricle is captured according to a difference value between the first delivery time (VP_OP) and the first sensing time (GS_OP) comprises:
if the difference value between the first delivery time (VP_OP) and the first sensing time (GS_OP) is a positive number and is less than a sixth threshold, determining the ventricle is captured, otherwise determining the ventricle is not captured.

16. The medical instrument of claim 1, wherein the control device is further configured to perform, during the operational period, the following steps:
acquiring a first delivery time (VP_OP); wherein the first delivery time (VP_OP) is the first specific time, and the first delivery time is time when a pacing pulse is delivered by the first electrode pair; and
acquiring the second in-vivo near-field myocardial electrogram by the second electrode pair and acquiring an eighth sensing time (LS2'_OP); wherein the eighth sensing time is the second specific time, and the eighth sensing time is a sensing time (LS2'_OP) of ventricular muscle activation corresponding to a pacing event in the second in-vivo near-field myocardial electrogram; and
the step of determining the origin of ventricular activation of the heartbeat according to the difference value between the first specific time and the second specific time comprises:
determining whether the ventricle is captured according to a difference value between the first delivery time (VP_OP) and the eighth sensing time (LS2'_OP).

17. The medical instrument of claim 16, wherein the step of determining whether the ventricle is captured from a difference value between the first delivery time (VP_OP) and the eighth sensing time (LS2'_OP) comprises:
if a ratio of a seventh difference value to an eighth difference value is greater than a seventh threshold, determining the ventricle is captured, otherwise determining the ventricle is not captured;
wherein the seventh difference value is a difference value between the first delivery time (VP_OP) and the eighth sensing time (LS2'_OP), and the eighth difference value is determined from a difference value between a fourth sensing time (LS1_SUP) and a sixth sensing time (LS2_SUP); and the sixth sensing time (LS2_SUP) is a sensing time corresponding to a sensed event in the second in-vivo near-field myocardial electrogram acquired by the second electrode pair within a set up period, the fourth sensing time (LS1_SUP) is a sensing time corresponding to a sensed event in the first in-vivo near-field myocardial electrogram acquired by the first electrode pair within the set up period, and the origin of ventricular activation of the heartbeat corresponding to the set up period is atrium originated ventricular activation.

18. The medical instrument of claim 16, wherein the step of determining whether the ventricle is captured from a difference value between the first delivery time and the eighth sensing time comprises:
if a difference value between a seventh difference value and a ninth difference value is less than an eighth threshold, determining the ventricle is captured, otherwise determining the ventricle is not captured;
wherein the seventh difference value is the difference value between the first delivery time (VP_OP) and the eighth sensing time (LS2'_OP), and the ninth difference value is determined from a difference value between a second delivery time (VP_SUP) and a ninth sensing time (LS2'_SUP); and the ninth sensing time (LS2'_SUP) is a sensing time of ventricular muscle activation corresponding to a pacing event in the second in-vivo near-field myocardial electrogram acquired by the second electrode pair when the ventricle is captured by pacing within a set up period, and the second delivery time (VP_SUP) is time when a pacing pulse is delivered through the first electrode pair when the ventricle is captured by pacing within the set up period.

19. The medical instrument of claim 1, wherein the control device is further configured to perform, during the operational period, the following steps:
acquiring a first sensing time (GS_OP); wherein the first sensing time is the first specific time, and the first sensing time is a sensing time of an R-wave in a far-field cardiac electrogram; and
acquiring the second in-vivo near-field myocardial electrogram by the second electrode pair and acquiring an eighth sensing time (LS2'_OP); wherein the eighth sensing time is the second specific time, and the eighth sensing time is a sensing time of ventricular muscle activation corresponding to a pacing event in the second in-vivo near-field myocardial electrogram; and
the step of determining the origin of ventricular activation of the heartbeat according to the difference value between the first specific time and the second specific time comprises:
determining whether the ventricle is captured from a difference value between the eighth sensing time (LS2'_OP) and the first sensing time (GS_OP).

20. The medical instrument of claim 19, wherein the step of determining whether the ventricle is captured from a difference value between the eighth sensing time (LS2'_OP) and the first sensing time (GS_OP) comprises:
if a difference value between a tenth difference value and an eleventh difference value is less than a ninth threshold, determining the ventricle is captured, otherwise determining the ventricle is not captured;
wherein the tenth difference value is the difference value between the eighth sensing time (LS2'_OP) and the first sensing time (GS_OP), and the eleventh difference value is determined according to a difference value between a ninth sensing time (LS2'_SUP) and a seventh sensing time (GS'_SUP); and the ninth sensing time (LS2'_SUP) is a sensing time of ventricular muscle activation corresponding to a pacing event in an in-vivo near-field myocardial electrogram acquired by the second electrode pair when the ventricle is captured by pacing within a set up period, and the seventh sensing time (GS'_SUP) is a sensing time of an R-wave in a far-field cardiac electrogram acquired when the ventricle is captured by pacing within the set up period.

21. The medical instrument of claim 19, wherein the step of determining whether the ventricle is captured according to a difference value between the eighth sensing time (LS2'_OP) and the first sensing time (GS_OP) comprises:
if a ratio of a tenth difference value to a twelfth difference value is greater than a tenth threshold, determining the ventricle is captured, otherwise determining the ventricle is not captured;
wherein the tenth difference value is the difference value between the eighth sensing time (LS2'_OP) and the first sensing time (GS_OP), and the twelfth difference value is determined from a difference value between a sixth sensing time (LS2_SUP) and a third sensing time (GS_SUP); and the sixth sensing time (LS2_SUP) is a sensing time corresponding to a sensed event in an in-vivo near-field myocardial electrogram acquired by the second electrode pair within a set up period, the third sensing time (GS_SUP) is a sensing time of an R-wave in a far-field cardiac electrogram acquired within a set up period, and the set up period corresponds to atrium originated ventricular activation.

22. The medical instrument of claim 1, wherein the control device is further configured to perform, during the operational period, the following step:
determining the origin of ventricular activation of the heartbeat according to a difference value between a first specific difference value and a second specific difference value or a ratio of a first specific difference value and a second specific difference value;
wherein the first specific difference value is the difference value between the first specific time and the second specific time, the second specific difference value is a difference value between a third specific time and a fourth specific time, and the third specific time and the fourth specific time are selected from the following times acquired within a set up period: sensing time (GS_SUP) of an R-wave in a far-field cardiac electrogram, sensing time (GS'_SUP) of an R-wave in a far-field cardiac electrogram acquired when the ventricle is captured by pacing, sensing time (LS2_SUP) corresponding to a sensed event in a second in-vivo near-field myocardial electrogram acquired by the second electrode pair, sensing time (LS2'_SUP) of ventricular muscle activation corresponding to a pacing event in a second in-vivo near-field myocardial electrogram acquired by the second electrode pair, sensing time (LS1_SUP) corresponding to a sensed event in a first in-vivo near-field myocardial electrogram acquired by the first electrode pair, and time (VP_SUP) when a pacing pulse is delivered by the first electrode pair during the ventricle is captured by pacing.

23. The medical instrument of any one of claims 12-21, wherein the control device being further configured to perform, during the operational period, the following step comprises:
after a first preset number of heartbeats within the operational period, the control device being configured to perform the following step:
for a second preset number of heartbeats, if it is determined that there are a third preset number of heartbeats as paced heartbeats, determining that the ventricle has been captured, otherwise determining that the ventricle is being captured;
wherein the third preset number is less than or equal to the second preset number.

24. The medical instrument of any one of claims 12-21, further comprising at least one ventricular electrode lead, the at least one ventricular electrode lead being further configured with the first electrode pair and/or the second electrode pair for sensing.

25. The medical instrument of any one of claims 12-21, wherein the first sensed event and/or the second sensed event is ventricular activation, and the first electrode pair and/or the second electrode pair is an electrode pair for attachment to a ventricle or an epicardium of the ventricle.
